# EUROPEAN PATENT APPLICATION

(11) **EP 4 374 808 A1**
(43) Date of publication of application: **29.05.2024**
(21) Application number: 22209744.6
(22) Date of filing: 25.11.2022
(51) Int. Cl.: A61B 18/08, A61B 18/14, A61F 2/95, A61B 17/3207

(54) **CATHETER ASSEMBLY FOR SLITTING A DISSECTION MEMBRANE OF AN AORTA**

(71) Applicant: Universität Linz, 4040 Linz (AT)
(72) Inventor: Zierer, Andreas,, 4040 Linz (AT)
(74) Representative: Vossius & Partner Patentanwälte Rechtsanwälte mbB

(57) **Abstract**

The invention relates to a catheter assembly for slitting a dissection membrane (4) of an aorta of a patient. The assembly comprises a first catheter (2) configured to be inserted into a true lumen (6) located on a first side of the dissection membrane (4), a second catheter (8) configured to be inserted into a false lumen (10) located on a second side of the dissection membrane (4), and an electrically conductive wire (12) configured to be arranged to be electrically conductively joined to the first catheter (2) and the second catheter (8). The wire (12) is further configured to be energized with electrical current such that the dissection membrane (4) can be slit by the wire (12). Also described is a method of slitting a dissection membrane (4) of an aorta of a patient, comprising the steps of (a) arranging a first catheter (2) to extend at least partially within a true lumen (6) of the aorta located on a first side of the dissection membrane (4), (b) arranging a second catheter (8) to extend at least partially within a false lumen (10) of the aorta located on a second side of the dissection membrane (4), (c) arranging an electrically conductive wire (12) so as to be electrically conductively joined to the first catheter (2) and the second catheter (8), (d) applying an electrical current to the wire (12); and (e) slitting the dissection membrane (4) by moving the wire (12) relative to the dissection membrane (4).

## Description

The present disclosure relates to a method of slitting a dissection membrane of an aorta and a catheter assembly for slitting a dissection membrane of an aorta.

### BACKGROUND

Aortic dissection occurs when an injury to the innermost layer of the aorta allows blood to flow between the layers of the aortic wall, forcing the layers apart. Aortic dissection can quickly lead to death from insufficient blood flow to the heart or complete rupture of the aorta. Two main types of aortic dissection are Stanford type A, which involves the first part of the aorta (adjacent to the heart), and type B, which does not.

The aorta is made up of three layers, the intima, the media, and the adventitia. The intima is in direct contact with the blood inside the vessel, and mainly consists of a layer of endothelial cells on a basement membrane; the media contains connective and muscle tissue, and the vessel is protected on the outside by the adventitia, comprising connective tissue. The initiating event in aortic dissection is a tear in the intimal lining of the aorta. Due to the high pressures in the aorta, blood enters the media at the point of the tear. The force of the blood entering the media causes the tear to extend. It may extend proximally (closer to the heart) or distally (away from the heart) or both. The blood travels through the media, creating a "false lumen". The "true lumen" is the normal conduit of blood in the aorta. Separating the false lumen from the true lumen is a layer of intimal tissue known as the intimal or dissection flap. The vast majority of aortic dissections originate with an intimal tear in either the ascending aorta (65%), the aortic arch (10%), or just distal to the ligamentum arteriosum in the descending thoracic aorta (20%).

Aortic dissection is one of the most feared diseases of the aorta. Treatment of post-dissection arch and thoracoabdominal aortic aneurysms presents significant therapeutic challenges. Despite improvements in diagnostics, medical therapy and surgical technique patient mortality and morbidity remain high.

Management of aortic dissection inter alia includes stenting the aorta, particularly under use of a stent graft.

Replacement of the ascending aorta with resection of the entry site leaves the downstream dissected aorta untouched. A residual dissection membrane remains with a patent false lumen. Due to the patent false lumen in the descending aortic segments a proportion of patients will develop post-dissections aneurysms. According to Evangelista et al. 2012, 9% of patients require further therapy during follow-up due to aortic dilation (Evangelista, A. et al.: Long-term outcome of aortic dissection with patent false lumen: predictive role of entry tear size and location. Circulation, 2012; 125(5): 3133-41).

Management of aortic arch and thoraco-abdominal dissecting aneurysm is complex and the best treatment option depends on many factors. Beside patient related factors, there are some technical, anatomical issues. The proximal sealing zone often involves the aortic arch. Specifically, there is a risk that the true lumen collapses or takes off of aortic branches from the false lumen such that an endograft alignment is difficult or even impossible. Open thoracoabdominal repair remains a formidable surgical challenge.

More specifically, a very narrow true lumen which often is only slit-shaped makes an endograft alignment difficult, particularly because of the risk of stent obstruction caused by a thick chronic dissection flap. Aortic branches can be perfused either by the true or the false lumen. These facts make a specific treatment concept for each patient mandatory. Particularly, if the true lumen is small, there is a risk that an endograft cannot be deployed to its intended size.

Therefore, there is a need for a further treatment method of an aortic dissection; specifically, there is a need for a method of slitting a dissection membrane of an aorta of a patient. Further, there is a need for a system for managing an aortic dissection; specifically, there is a need for a catheter assembly for slitting a dissection membrane of an aorta of a patient.

These objects are achieved by the independent claims. Dependent claims refer to preferred embodiments. Additional and/or alternative aspects of the present disclosure are discussed in the specification and in the aspects.

### SUMMARY

According to the present disclosure a method of slitting a dissection membrane of an aorta of a patient is provided. The method comprises the following steps (a) arranging a first catheter to extend at least partially within a true lumen of the aorta located on a first side of the dissection membrane, (b) arranging a second catheter to extend at least partially within a false lumen of the aorta located on a second side of the dissection membrane, (c) arranging an electrically conductive wire so as to be electrically conductively joined to the first catheter and the second catheter, (d) applying an electrical current to the wire; and (e) slitting the dissection membrane by moving the wire relative to the dissection membrane.

The method may be carried out under use of a catheter assembly for slitting a dissection membrane of an aorta of a patient as described herein. A catheter assembly for slitting a dissection membrane of an aorta of a patient according to the present description comprises a first catheter, a second catheter, and an electrically conductive wire.

The first catheter is configured to be inserted into a true lumen located on a first side of the dissection membrane. The second catheter is configured to be inserted into a false lumen located on a second side of the dissection membrane. The electrically conductive wire is configured to be arranged to be electrically conductively joined to the first catheter and the second catheter, wherein the wire is further configured to be energized with electrical current such that the dissection membrane can be slit by the wire.

By slitting the dissection membrane, the true lumen of the aorta can be enlarged such that subsequently an implantation of an endovascular stent graft is enabled or facilitated. Specifically, a common lumen for an endovascular procedure, particularly for an endograft placement can be achieved by the method. In other words, a large communication can be created between the true lumen and the false lumen. This so-called "new lumen" or "common lumen" has the potential to make an endograft implantation feasible and to ensure perfusion of the aortic branches and organs of the patient.

The method and the assembly are particularly effective and safe to deal with the problem of a narrow true lumen of post-dissection aneurysm. It generally allows slitting the dissection membrane from the aortic arch down to the iliac bifurcation.

The method and the assembly constitute an effective approach to eliminate previous contradictions for thoracoabdominal endovascular repair such as compressed true lumen or take off of aortic branches from the false lumen.

The method can, but does not have to, be performed with a surgically opened thorax. Also, the method can, but does not have to, be performed during open aortic arch replacement, e.g., employing the frozen elephant trunk technique.

Various embodiments may implement the following features:
The first catheter and the second catheter may be made from an electrically insulating material.

The first catheter may have a length that is between 1 m and 2 m, preferably between 1 m and 1.5 m. The second catheter may have a length that is between 1 m and 2 m, preferably between 1 m and 1.5 m. The first catheter may have a first end configured to be arranged outside the body of the patient and an opposing second end configured to be arranged within the body of the patient. The second catheter may have a first end configured to be arranged outside the body of the patient and an opposing second end configured to be arranged within the body of the patient.

The first catheter may have a lumen extending between its first end and its second end. The second catheter may have a lumen extending between its first end and its second end. In step (c), the wire may be arranged such that it extends partly within the lumen of the first catheter and partly within the lumen of the second catheter. In step (c), the wire may be arranged such that a first end of the wire protrudes from the first end of the first catheter and such that a second end of the wire opposite the first end of the wire protrudes from the first end of the second catheter.

In step (a), the first catheter may be inserted via a first groin of the patient. In step (b), the second catheter may be inserted via the second groin of the patient.

The catheters may be configured so that a contrast medium can be brought to the dissection membrane by passing through them, for example by passing through the lumens of the catheters. This allows facilitated control of the method using X-ray.

The wire may have a length that is between 1.5 m and 4 m, preferably between 2 m and 3.5 m.

The wire may comprise a central region configured to contact the dissection membrane for slitting the dissection membrane. The central region may be located so that it is less than 40 cm from the geometric center of the wire, preferably less than 30 cm, preferably less than 20 cm. The central region may have an extension measured along the longitudinal extension of the wire that is between 1 cm and 10 cm, preferably between 2 cm and 5 cm.

In step (c), the central region may be arranged between the second end of the first catheter and the second end of the second catheter.

The wire may comprise an electrically insulating sheath and, in its central region, an electrically non-insulated region for slitting the dissection membrane. The non-insulated region may be surrounded, preferably directly surrounded by the insulating sheath. The non-insulated region may be the only non-insulated region of the central region of the wire. In this way, the current applied to the wire in step (d) can be confined to the non-insulated region.

The non-insulated region may extend along a portion of the longitudinal extension of the wire. The non-insulated region may extend only over a portion of the circumference of the wire.

The central region may comprise a preformed kink. The preformed kink may be located between two opposing ends of the non-insulated region. The kink may be configured so that the wire forms two legs through the kink that enclose an angle between 45° and 135° when no other forces (except gravitation) act on the wire, preferably an angle between 60° and 120°, preferably between 60° and 90°. The kink facilitates proper positioning of the wire vis-à-vis the dissection membrane, particularly during performance of step (e).

In step (e), the first catheter and the second catheter may be each moved in a distal direction, particularly with the wire fixed in its longitudinal position relative to the first and second catheter. To this end, the catheter arrangement may further comprise means for fixing the wire relative to the first catheter and/or relative to the second catheter.

In this way, a movement of the wire, particularly of its central region relative to the first and/or second catheter during step (e) can be avoided. This reduces the risk of unwanted injury to body tissue particularly during step (e). Thus, the slitting process can be performed in a particularly safe and controlled manner.

The electrical current applied to the wire in step (d) may be configured to heat the wire such that the slitting during step (e) may be carried out by electrocauterization. Correspondingly, step (d) may be carried out at least during step (e), for example exclusively during step (e).

The wire may have a first end and/or a second end that is configured to be connected to an electrical current source for applying the electrical current to the wire, particularly to the non-insulated region of the wire. To this end, the catheter assembly may comprise a corresponding electrical current source. The current may have an electric voltage between 100 V and 300 V. The current may be an alternating current having a frequency between 200 kHz and 600 kHz. The electrical current source may comprise a radiofrequency generator.

Step (d) may comprise connecting the wire to the electrical current source by providing an electrical connection between the first end and/or second end of the wire that is configured to be connected to an electrical current source and the electrical current source.

In step (d), the current source may be electrically connected to the wire via a connector. The connector may be mechanically and electrically connected to the corresponding first end and/or second end of the wire. The current source may be connected to a handle, particularly to an electrocautery handle that is configured to be electrically connected to the connector to provide the wire with electrical current generated by the current source.

Expediently, step (a) and/or step (b) and/or step (c) and/or step (e) is or are performed under X-ray control. This allows the method to be carried out in a particularly suitable controlled manner.

The method may further comprise the following step: (f) inserting the wire through one of the two catheters, particularly through the lumen of one of the two catheters until an end portion of the wire is outside the one of the two catheters, inserting an auxiliary wire through the other of the two catheters, and grasping the end portion of the wire with the auxiliary wire, and pulling the wire with the auxiliary wire toward the other of the two catheters. In this way, the method can be suitably carried out without having to surgically open the thorax of the patient.

To this end, the wire may comprise an end portion provided with a hook. Correspondingly, the catheter assembly may further comprise an auxiliary wire for gripping the end portion of the wire. The auxiliary wire may comprise an end portion provided with an eyelet, the eyelet being configured for gripping the hook of the end portion of the wire. This facilitates performing step (f), particularly grasping or snaring the end portion of the wire with the auxiliary wire in step (f).

Particularly, step (f) may be carried out in a state in which the first catheter is arranged in the true lumen and the second catheter is arranged in the false lumen.

Inserting the wire through the lumen of one of the two catheters until an end portion of the wire is outside the one of the two catheters in step (f) may be carried out by inserting the wire into the first end of the one of the two catheters and pushing the wire through the lumen of the one of the two catheters until the end portion of the wire protrudes from the second end of the one of the two catheters.

Step (f) may further comprise pulling the end portion of the wire with the auxiliary wire through the second end of the other of the two catheters into the lumen of the other of the two catheters until the end portion of the wire protrudes from the first end of the other of the two catheters such that it can be grasped.

Step (f) may be carried out under X-ray control.

The first catheter and/or the second catheter may respectively comprise a protrusion region extending radially beyond a remaining region of the catheter. The protrusion region allows, on the one hand, the wire to be easily placed suitably close to the dissection membrane so that the slitting operation can be suitably carried out and, on the other hand, the wire to maintain a certain desired distance from wall areas of the aorta during the slitting operation, so that the risk of unwanted tissue damage is thereby significantly reduced. Moreover, the desired distance from wall areas can be kept quasi constant throughout the slitting process in step (e).

The protrusion region may be formed by a balloon.

The protrusion region preferably is formed eccentric with respect to the remaining region of the respective catheter. For example, the protrusion region may be configured such that a major axis defined by the protrusion region, or a center of the protrusion region is at a distance from a major axis defined by the remaining region of the catheter. In this way, the desired distance from wall areas of the aorta during the slitting operation can be suitable maintained.

The protrusion region may be configured to be filled with a contrast medium. This facilitates controlled movement of the catheters, particularly movement under X-ray control.

The respective catheter may further comprise a second lumen configured such that a contrast medium can be filled, for example pushed into the protrusion region by the second lumen.

The protrusion preferably is a three-dimensional body extending circumferentially around the catheter.

For example, the protrusion region may comprise two or more chambers configured to provide the eccentricity of the protrusion region.

The eccentricity may be such, that the protrusion extends less beyond the catheter at a side to face/facing the dissection membrane than to a side to face/facing away from the dissection membrane. The ratio of the eccentricity is not limited and depends on the anatomic and/or spatial situation. It may range between one third and two thirds. The eccentricity may be variable and/or can be individually adapted.

The balloon may be an inflatable balloon. Thus, step (a) of the method may further comprise arranging the first catheter with its balloon deflated within the true lumen and subsequently inflating the balloon. Correspondingly, step (b) of the method may further comprise arranging the second catheter with its balloon deflated within the false lumen and subsequently inflating the balloon. Preferably step (e) is carried out with the balloons of both catheters inflated.

According to a further aspect of the description, a use of a catheter assembly as described herein for slitting a dissection membrane of an aorta of a patient is provided.

### ASPECTS

In particular, the present disclosure comprises the following aspects:
1. A method of slitting a dissection membrane (4) of an aorta of a patient, comprising the steps of
   (a) arranging a first catheter (2) to extend at least partially within a true lumen (6) of the aorta located on a first side of the dissection membrane (4)
   (b) arranging a second catheter (8) to extend at least partially within a false lumen (10) of the aorta located on a second side of the dissection membrane (4),
   (c) arranging an electrically conductive wire (12) so as to be electrically conductively joined to the first catheter (2) and the second catheter (8),
   (d) applying an electrical current to the wire (12); and
   (e) slitting the dissection membrane (4) by moving the wire (12) relative to the dissection membrane (4).
2. Method according to aspect 1, wherein in step (e) the wire (12) is held fixed relative to the first catheter (2) and/or relative to the second catheter (8), particularly held fixed with respect to the longitudinal extension of the wire (12).
3. Method according to aspect 1 or 2, wherein for applying electric current to the wire (12), in step (d), a current source (52), for example comprising a radiofrequency generator, is electrically connected to the wire (12) via a connector (16).
4. Method according to aspect 3, wherein the current source (52) is electrically connected to a cautery handle (50) that is configured to be electrically connected to the connector (16).
5. Method according to any of the preceding aspects, wherein the current has an electric voltage between 100 V and 300 V,
6. Method according to any of the preceding aspects, wherein the current is an alternating current having a frequency between 200 kHz and 600 kHz.
7. Method according to any of the preceding aspects, wherein
   in step (a) the first catheter (2) is inserted via a first groin of the patient, preferably percutaneously inserted.
8. Method according to aspect 7, wherein
   in step (b), the second catheter (8) is inserted via the second groin of the patient, preferably percutaneously.
9. Method according to any of the preceding aspects, wherein in step (e) the first catheter (2) and the second catheter (4) are each moved in a distal direction.
10. Method according to any of the preceding aspects, wherein step (a) and/or step (b) and/or step (c) and/or step (e) is or are performed under X-ray control.
11. Method of any of the preceding aspect, wherein a contrast medium is pushed through the first catheter (2) and/or the second catheter (8) to the dissection membrane (4).
12. Method according to any of the preceding aspects, further comprising the following step.
   (f) inserting the wire (12) through one of the two catheters (2, 8) until an end portion (122) of the wire (12) is outside the one of the two catheters (2, 8),
   inserting an auxiliary wire (14) through the other of the two catheters (8, 2), and
   grasping the end portion (122) of the wire (12) with the auxiliary wire (14) and pulling the wire (12) with the auxiliary wire (14) toward the other of the two catheters (8, 2).
13. A catheter assembly for slitting a dissection membrane of an aorta of a patient, comprising
   a first catheter (2) for insertion into a true lumen (6) located on a first side of the dissection membrane (4),
   a second catheter (8) for insertion into a false lumen (10) located on a second side of the dissection membrane (4), and
   an electrically conductive wire (12) configured to be arranged to be electrically conductively joined to the first catheter (2) and the second catheter (8),
   wherein the wire (12) is further configured to be energized with electrical current such that the dissection membrane (4) can be slit by the wire (12).
14. Catheter assembly according to aspect 13, wherein the wire (12) comprises a central region (128) configured to contact the dissection membrane (4) for slitting the dissection membrane (4).
15. Catheter assembly according to aspect 14, wherein the wire (12) comprises an electrically insulating sheath (124) and, in the central region (128), an electrically non-insulated region (126) for slitting the dissection membrane (4).
16. Catheter assembly according to aspect 14 or 15, wherein the central region (128) comprises a preformed kink.
17. Catheter arrangement according to any of aspects 13 to 16, wherein the first catheter (2) and/or the second catheter (8) respectively comprises a protrusion region (30) extending radially beyond a remaining region of the catheter (2, 8).
18. Catheter arrangement according to aspect 17, wherein the protrusion region (30) is formed by a balloon, preferably by an inflatable balloon and/or, preferably, the protrusion region is eccentrically arranged with regard to the major axis (HK) of the catheter.
19. Catheter arrangement according to aspect 17 or 18, wherein the protrusion region (30) is configured such that a major axis (HB) defined by the protrusion region (30), or a center of the protrusion region (30) is at a distance (δ) from a major axis (HK) defined by the remaining region of the catheter (2, 8).
20. Catheter arrangement according to any of aspects 17 to 19, wherein the protrusion region (30) is configured to be filled with a contrast medium.
21. Catheter assembly according to any of aspects 17 to 20, wherein the wire (12) is electrically connected to a connector (16).
22. Catheter assembly according to aspect 21, wherein the wire (12) is electrically connected to a power source (22) via the connector (16).
23. Catheter arrangement according to any of aspects 13 to 22, further comprising means for fixing the wire (12) relative to the first catheter (2) and/or relative to the second catheter (8).
24. Catheter assembly according to any of aspects 13 to 23, wherein the wire (12) comprises an end portion (122) provided with a hook.
25. Catheter assembly according to aspect 24, further comprising an auxiliary wire (14) for gripping the end portion (122) of the wire (12), the auxiliary wire (14) preferably comprising an end portion (142) provided with an eyelet, the eyelet being configured for gripping the hook of the end portion (122) of the wire (12).
26. Use of a catheter assembly according to any of aspects 13 to 25 for slitting a dissection membrane (4) of an aorta of a patient.

### SHORT DESCRIPTION OF THE DRAWINGS

The subject-matter of the disclosure will be explained in more detail with reference to preferred exemplary embodiments which are illustrated in the attached drawings, in which:
Figures 1a to 1c are schematic views illustrating steps of a method of slitting a dissection membrane of an aorta of a patient according to the present description.
Fig. 2 is a schematic cross section of a portion of a catheter and a wire used in the method.
Fig. 3a is a schematic cross-section of a catheter taken at a location of a protrusion region of the catheter.
Fig. 3b is a schematic cross-section of a first catheter and a second catheter and a wire during a step of slitting the dissection membrane.
Fig. 4 is a schematic view of the wire, provided at one end with a connector, and a cautery handle to be connected to the connector, the cautery handle connected to an electrical current source.
Fig. 5 is a schematic view of a process of grasping or snaring an end portion of the wire with an auxiliary wire.

### DETAILED DESCRIPTION

Herein, a method for treating an aortic dissection is described. The method preferably comprises an endovascular stenting of the aorta, for example by inserting a stent graft. Prior to the endovascular stenting, the dissection membrane of the aorta is slitted according to a corresponding method as described herein. Slitting the dissection membrane is suitable for enlarging the true lumen of the dissected membrane of the aorta. In this way, subsequent implantation of an endovascular stent graft is enabled or facilitated.

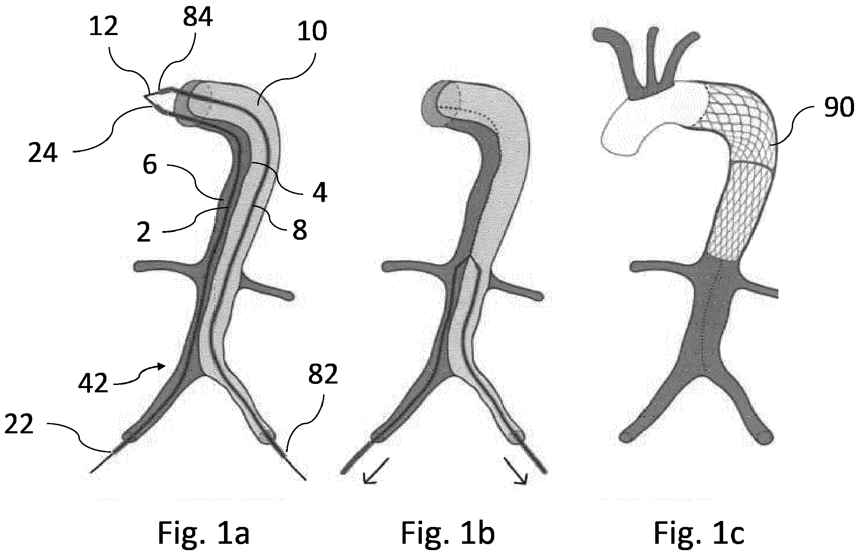

Figures 1a to 1c are schematic views illustrating steps of a method of slitting a dissection membrane of an aorta of a patient according to the present description. Fig. 1a schematically illustrates an aorta of a patient. The aorta has a dissection membrane 4. On a first side of the dissection membrane 4 is a true lumen 6 and on a second side of the dissection membrane 4 is a false lumen 10. In the exemplary sketch of Fig. 1a, the false lumen 10 extends distally beyond the bifurcation 42 of the aorta. This is, however, merely one example. Generally, there are many different possible forms of the dissection membrane 4, the true lumen 6 and the false lumen 10.

The method of slitting a dissection membrane comprises the following step (a): Arranging a first catheter 2 to extend at least partially within the true lumen 6 of the aorta located on the first side of the dissection membrane 4. The method further comprises the following step (b): Arranging a second catheter 8 to extend at least partially within the false lumen 10 of the aorta located on the second side of the dissection membrane 4.

The first catheter 2 has a first end 22 configured to be arranged outside the body of the patient and an opposing second end 24 configured to be arranged within the body of the patient. The second catheter 8 has a first end 82 configured to be arranged outside the body of the patient and an opposing second end 84 configured to be arranged within the body of the patient.

The first catheter 2 has a lumen extending between its first end 22 and its second end 24. The second catheter 8 has a lumen extending between its first end 82 and its second end 84.

In step (a), the first catheter 2 may be inserted percutaneously via a first groin of the patient, and in step (b), the second catheter 8 may be inserted percutaneously via the second groin of the patient.

In step (a), the first catheter 2 may be arranged such that its second end 24 is located within a region of the aorta where the false lumen 10 and true lumen 6 are directly connected, i. e. where there is no membrane between the two lumens 6, 10.

In step (b), the second catheter 8 may be arranged such that its second end 84 is located within said region of the aorta.

The method further comprises the following step (c): Arranging an electrically conductive wire 12 so as to be electrically conductively joined to the first catheter 2 and the second catheter 8. Specifically, the wire 12 may be arranged in step (c) so as to extend through the lumen of the first catheter 2 and the lumen of the second catheter 8.

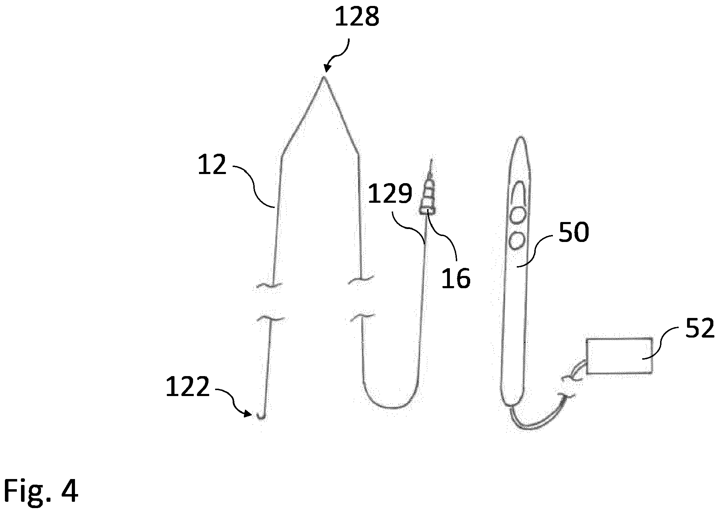

The method further comprises the following step (d): applying an electrical current to the wire 12. Specifically, as sketched in Fig. 4, for applying electric current to the wire 12, in step (d), a current source 52 may be electrically connected to the wire 12 via a connector 16. For example, the connector 16 may be mechanically and electrically connected to an end or end section 129 of the wire 12. The current source 52 may be connected electrically to a cautery handle 50 that is configured to be electrically and mechanically coupled to the connector 16.

The current may have an electric voltage between 100 V and 300 V. The current may be an alternating current having a frequency between 200 kHz and 600 kHz.

The method further comprises the following step (e): slitting the dissection membrane 4 by moving the wire 12 to which the current has been applied in step (d) relative to the dissection membrane 4. The wire 12 and the current may be configured such that the slitting of the dissection membrane 4 can be carried out by an electrocautery process.

In step (e), the wire 12 is preferably held fixed relative to the first catheter 2 and the second catheter 8. For example, a first means, e. g. in form of a clamp, may be provided to fix the wire 12 with respect to its longitudinal extension relative to the first end 22 of the first catheter 2. Correspondingly, a second means, e. g. in form of a further clamp, may be provided to fix the wire 12 with respect to its longitudinal extension relative to the first end 82 of the second catheter 8.

In step (e) the first catheter 2 and the second catheter 4 may each be moved in a distal direction, as exemplarily sketched by arrows in Fig. 1b. In this way, the slitting process can be carried out in a in particularly suitable controlled manner.

Fig. 1b schematically illustrates a state where the catheters 2, 8 have been moved or pulled distally a certain distance.

Fig. 1c schematically illustrates the aorta after having inserted an endovascular stent graft 90 in the aorta for stabilizing the vessel. In general, a stent or stent graft can be inserted more easily into a vessel with a larger lumen than into a vessel with a smaller lumen. By slitting the dissection membrane 4, a comparatively large lumen can be generated, particularly a lumen that is larger than both the true lumen and the false lumen. Therefore, the stent graft 90 can be inserted in a particularly suitable manner subsequently to the slitting of the dissection membrane 4.

Preferably, step (a) and/or step (b) and/or step (c) and/or step (e) are performed under X-ray control. For example, a contrast medium may be applied to the dissection membrane 4 using the lumens of the catheters 2, 8. Accordingly, the lumens of the catheters are preferably configured to be filled with contrast medium.

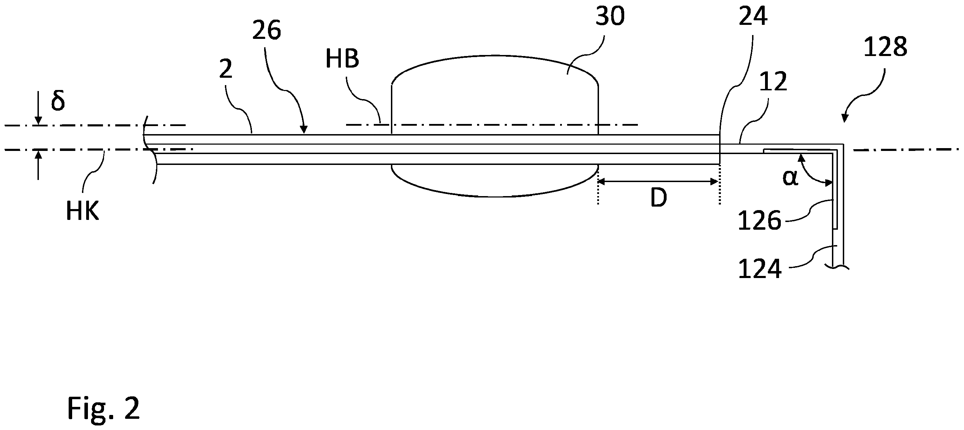

As schematically illustrated in Fig. 2, the wire 12 comprises a central region 128 configured to contact the dissection membrane 4 for slitting the dissection membrane 4. Preferably, the central region 128 comprises a preformed kink. In this way, the wire 12 can be suitably positioned vis-à-vis the dissection membrane 4 prior to and during step (e).

The kink may be configured so that the wire 12 forms two legs through the kink that enclose an angle α preferably between 60° and 120°, for example between 80° and 100° when no other forces (except gravitation) act on the wire 12. The kink is preferably configured such that it can be straightened out in order to pull or push the wire 12 through at least one of the lumens of the catheters 2, 8.

The wire 12 preferably comprises an electrically insulating sheath 124 and, in the central region 128, an electrically non-insulated region 126 for slitting the dissection membrane 4. The non-insulated region 126 may be an elongated region extending along the longitudinal extension of the wire 12. The length of the non-insulated region 126 may be between 0.5 cm and 4 cm, preferably between 1 cm and 3 cm.

Preferably, the first catheter 2 and/or the second catheter 8 respectively comprises a protrusion region 30 extending radially beyond the remaining region of the catheter 2, 8. This is exemplarily sketched in Fig. 2 for the first catheter 2. The protrusion region 30 may be formed by a balloon, particularly by an inflatable balloon.

Preferably, the configuration is such that the balloon 30 can be inflated via the respective catheter 2, 8. To this end the respective catheter 2, 8 may comprise a further lumen extending from its first end 22, 82 to the corresponding balloon 30.

The protrusion region or balloon 30 is preferably configured such that it protrudes radially beyond a circumferential surface 26 of the remaining region or portion of the respective catheter 2, 8 in an eccentric manner. For example, a major axis HB defined by the protrusion region 30, or a center of the protrusion region 30 may be at a distance δ from a major axis HK defined by the remaining region of the respective catheter 2, 8. The distance δ may be for example between 0.5 cm and 1 cm.

The protrusion 30 may have a radial extension between 0.5 cm and 3 cm, for example between 1 cm and 2.5 cm.

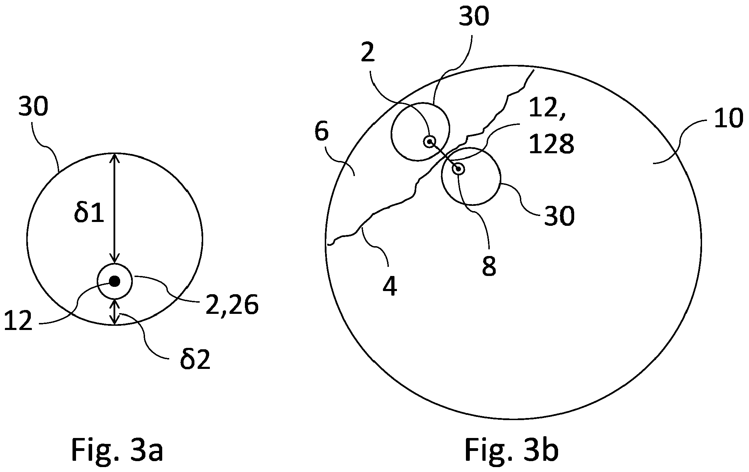

Fig. 3a shows a schematic cross-section of the catheter 2 in the area of the balloon 30. In the cross-section, a radially facing surface of the balloon 30 may have a maximum distance δ1 and a minimum distance δ2 to the circumferential surface 26 of the remaining region of the respective catheter 2, 8. The relation δ1 : δ2 may be between 20 : 1 and 1.5 : 1, preferably between 10 : 1 and 2 : 1.

Due to the eccentric design of the protrusion or balloon 30, the wire 12 can be advantageously positioned in relation to the dissection membrane 4 during the slitting process, i. e. during step (e) in such a way that the risk of the wire 12 undesirably contacting a tissue that is not intended for slitting is reduced, such as portions of walls of the aorta. This is schematically illustrated in Fig. 3b which shows the catheters 2, 8, the wire 12 and the dissection membrane 4 in a cross-section. Accordingly, the protrusions 30 ensures that the central region 128 of the wire 12, particularly the non-insulated region 126 maintains a certain minimum distance to tissue areas surrounding the dissection membrane 4. Preferably, the protrusion 30 has a distance D from the second end 24, 84 of the respective catheter 2, 8 that is between 1 cm and 5 cm, preferably between 1 cm and 3 cm. The protrusion 30 may have a maximum radial extension, wherein a distance between the maximum radial extension and the respective end of the catheter is between 1 cm and 4 cm. The maximum radial extension may be between 0.5 cm and 3 cm.

Preferably, the balloons 30 can be inflated and deflated. Preferably, the configuration is such that the catheters 2, 8 can be inserted into the body of the patient with the balloons 30 deflated, and such that the balloons 30 can be inflated when the catheters 2, 8 are arranged in the true lumen 6 and the false lumen 10 respectively, ready for starting step (e). This facilitates inserting the catheters 2, 8.

Preferably, the balloons 30 are configured to be filled with contrast medium. This further facilitates controlled movement under X-ray.

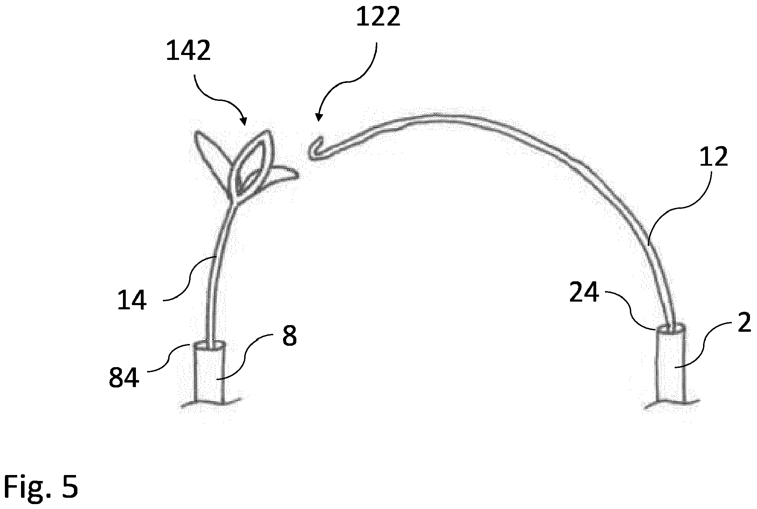

As exemplarily illustrated in Fig. 5, the method preferably further comprises the following step: (f) inserting the wire 12 through one of the two catheters 2, 8 (e. g. of the first catheter 2 as illustrated in Fig. 5) until an end portion 122 of the wire 12 is outside the one of the two catheters 2, 8, inserting an auxiliary wire 14 through the other of the two catheters 8, 2 (e. g. the second catheter 8 as illustrated in Fig. 5) and grasping the end portion 122 of the wire 12 with the auxiliary wire 14, and pulling the wire 12 with the auxiliary wire 14 toward the other of the two catheters 8, 2. Step (f) is preferably carried out after step (a) and step (b) and prior to or within step (c).

As sketched schematically in Figures 4 and 5, the end portion 122 is preferably provided with a hook. The auxiliary wire 14 preferably comprises an end portion 142 provided with an eyelet that is configured for gripping or snaring the hook of the end portion 122 of the wire 12. This configuration facilitates grasping the end portion 122 of the wire 12 with the auxiliary wire 14 in step (f).

Further according to the method, after carrying out step (e), a step (g) may follow in which the wire 12 is pulled out of the catheters 2, 8. Subsequently, the catheters 2, 8 may be removed from the body of the patient.

## Claims

1. A method of slitting a dissection membrane (4) of an aorta of a patient, comprising the steps of
(a) arranging a first catheter (2) to extend at least partially within a true lumen (6) of the aorta located on a first side of the dissection membrane (4)
(b) arranging a second catheter (8) to extend at least partially within a false lumen (10) of the aorta located on a second side of the dissection membrane (4),
(c) arranging an electrically conductive wire (12) so as to be electrically conductively joined to the first catheter (2) and the second catheter (8),
(d) applying an electrical current to the wire (12); and
(e) slitting the dissection membrane (4) by moving the wire (12) relative to the dissection membrane (4).

2. Method according to claim 1, wherein in step (e) the wire (12) is held fixed relative to the first catheter (2) and/or relative to the second catheter (8).

3. Method according to claim 1 or 2, wherein for applying electric current to the wire (12), in step (d), a current source (52) is electrically connected to the wire (12) via a connector (16), and/or
wherein the current has an electric voltage between 100 V and 300 V and/or wherein the current is an alternating current having a frequency between 200 kHz and 600 kHz.

4. Method according to any of the preceding claims, wherein
in step (a) the first catheter (2) is inserted via a first groin of the patient, and/or
in step (b), the second catheter (8) is inserted via the second groin of the patient.

5. Method according to any of the preceding claims, wherein in step (e) the first catheter (2) and the second catheter (4) are each moved in a distal direction, and/or
wherein step (a) and/or step (b) and/or step (c) and/or step (e) is or are performed under X-ray control.

6. Method according to any of the preceding claims, further comprising the following step.
(f) inserting the wire (12) through one of the two catheters (2, 8) until an end portion (122) of the wire (12) is outside the one of the two catheters (2, 8),
inserting an auxiliary wire (14) through the other of the two catheters (8, 2), and
grasping the end portion (122) of the wire (12) with the auxiliary wire (14) and pulling the wire (12) with the auxiliary wire (14) toward the other of the two catheters (8, 2).

7. A catheter assembly for slitting a dissection membrane of an aorta of a patient, comprising
a first catheter (2) configured to be inserted into a true lumen (6) located on a first side of the dissection membrane (4),
a second catheter (8) configured to be inserted into a false lumen (10) located on a second side of the dissection membrane (4), and
an electrically conductive wire (12) configured to be arranged to be electrically conductively joined to the first catheter (2) and the second catheter (8),
wherein the wire (12) is further configured to be energized with electrical current such that the dissection membrane (4) can be slit by the wire (12).

8. Catheter assembly according to claim 7, wherein the wire (12) comprises a central region (128) configured to contact the dissection membrane (4) for slitting the dissection membrane (4), and/or
wherein the wire (12) comprises an electrically insulating sheath (124) and, in the central region (128), an electrically non-insulated region (126) for slitting the dissection membrane (4), and/or
wherein the central region (128) comprises a preformed kink.

9. Catheter arrangement according to claim 7 or 8, wherein the first catheter (2) and/or the second catheter (8) respectively comprises a protrusion region (30) extending radially beyond the catheter (2, 8),
wherein preferably the protrusion region (30) is formed by a balloon.

10. Catheter arrangement according to claim 9, wherein the protrusion region (30) is configured such that a major axis (HB) defined by the protrusion region (30), or a center of the protrusion region (30) is at a distance (δ) from a major axis (HK) defined by the remaining region of the catheter (2, 8).

11. Catheter arrangement according to claim 9 or 10, wherein the protrusion region (30) is eccentrically arranged with regard to the major axis (HK) of the catheter.

12. Catheter assembly according to any claims 7 to 11, wherein the wire (12) is electrically connected to a connector (16),
wherein preferably the wire (12) is electrically connected to a power source (22) via the connector (16).

13. Catheter arrangement according to any of claims 7 to 12, further comprising means for fixing the wire (12) relative to the first catheter (2) and/or relative to the second catheter (8).

14. Catheter assembly according to any of claims 7 to 13, wherein the wire (12) comprises an end portion (122) provided with a hook,
wherein preferably the catheter assembly further comprises an auxiliary wire (14) for gripping the end portion (122) of the wire (12), the auxiliary wire (14) preferably comprising an end portion (142) provided with an eyelet, the eyelet being configured for gripping the hook of the end portion (122) of the wire (12).

15. Use of a catheter assembly according to any of claims 7 to 14 for slitting a dissection membrane (4) of an aorta of a patient.
